# EUROPEAN PATENT SPECIFICATION

(11) **EP 3 747 909 B1**
(45) Date of publication and mention of the grant of the patent: **04.03.2026**
(21) Application number: 19748189.8
(22) Date of filing: 30.01.2019
(51) Int. Cl.: C07K 14/00, C07C 255/00, A61K 31/513, A61K 31/704, A61K 9/00, A61K 33/243, A61K 45/06, A61K 47/42, A61K 47/60, A61K 47/64, A61P 35/00, C07K 5/02, C12N 9/99, A61K 38/00

(54) **ANTICANCER AGENT**
ANTIKREBSMITTEL
AGENT ANTICANCÉREUX

(30) Priority: 30.01.2018 JP 2018013678
(43) Date of publication of application: 09.12.2020
(73) Proprietor: Osaka University, Suita-shi, Osaka 565-0871 (JP)
(72) Inventor: MORI, Masaki, Suita-shi, Osaka 565-0871 (JP); ISHII, Hideshi, Suita-shi, Osaka 565-0871 (JP); KONNO, Masamitsu, Suita-shi, Osaka 565-0871 (JP); EGUCHI, Hidetoshi, Suita-shi, Osaka 565-0871 (JP); HARAGUCHI, Naotsugu, Suita-shi, Osaka 565-0871 (JP); TOSHIYAMA, Reishi, Suita-shi, Osaka 565-0871 (JP); NISHIYAMA, Nobuhiro, Tokyo 152-8550 (JP); TAKEMOTO, Hiroyasu, Tokyo 152-8550 (JP)
(74) Representative: Müller-Boré & Partner Patentanwälte PartG mbB
(86) International application number: PCT/JP2019/003150
(87) International publication number: WO 2019/151320

(56) References cited:
- EP-A1- 2 258 397
- WO-A1-2013/024048
- WO-A1-2017/170845
- WO-A2-2010/005740
- JP-A- 2002 515 418
- JP-A- 2017 105 800
- YAMASHITA, MASAFUMI ET AL.: "A CD 13 inhibitor, ubenimex, synergistically enhances the effects of anticancer drugs in hepatocellular carcinoma", INTERNATIONAL JOURNAL OF ONCOLOGY, vol. 49, 2016, pages 89 - 98, XP055628479
- ZHOU, ZHUXIAN ET AL.: "A multifunctional PEG-PLL drug conjugate forming redox-responsive nanoparticles for intracellular drug delivery", JOURNAL OF MATERIALS CHEMISTRY B, vol. 3, 2015, pages 7594 - 7603, XP055628483
- TOSHIYAMA, REISHI ET AL.: "Poly(ethylene glycol)- poly(lysine) block copolymer-ubenimex conjugate targets aminopeptidase N and exerts an antitumor effect in hepatocellular carcinoma stem cells", ONCOGENE, vol. 38, no. 2, August 2018 (2018-08-01), pages 244 - 260, XP036668959

## Description

### Technical Field

The present invention relates to an anticancer agent.

### Background Art

Ubenimex is known to bind to CD13/APN, which is present on the cell membrane of macrophages, to stimulate immunity. Ubenimex is a drug used in remission maintenance therapy for adult acute leukemia. Further, in recent years, ubenimex has been reported to inhibit the activity of CD13/APN in some solid cancers as well.

However, ubenimex cannot exert an effective anticancer effect on solid cancer unless it is administered at a concentration much higher than that used for acute leukemia. The IC₃₀ of ubenimex for hepatocellular carcinoma cell lines (HuH7, PLC) is 394.8 ug/ml for HuH7, and 498.8 ug/ml for PLC (Non-patent Literature (NPL) 1). In contrast, the dose of ubenimex for the aforementioned acute leukemia is 30 mg once a day in oral administration, and the maximum blood concentration achieved thereby is 2.21 ug/ml. Accordingly, it is speculated that when ubenimex is applied to a solid cancer, such as hepatocellular carcinoma, an effective anticancer effect cannot be achieved unless ubenimex is used at a concentration that is several hundred times as high as the blood concentration clinically obtained in the treatment of acute leukemia.
Further, Patent Literature (PL 1) describes a polymeric hyperbranched carrier-linked prodrug, and PL 2 describes a polymer conjugate of a physiologically active substance.

### Citation List

### Non-patent Literature

NPL 1: Yamashita M, Wada H, Eguchi H, Ogawa H, Yamada D, Noda T, et al. A CD13 inhibitor, ubenimex, synergistically enhances the effects of anticancer drugs in hepatocellular carcinoma. Int J Oncol, 2016; 49: 89-98.

### Patent Literature

PL 1: WO 2013/024048 A1PL 2: EP 2 258 397 A1

### Summary of Invention

### Technical Problem

An object to be achieved by the present invention is to provide a technique for enhancing the anticancer effect of ubenimex; in particular, enhancing its anticancer effect on solid cancer.

### Solution to Problem

As a result of extensive research in view of the above object, the present inventors found that the anticancer effect of ubenimex, especially its anticancer effect on solid cancer, can be enhanced by linking a plurality of ubenimex molecules to a chain polymer as outlined below. The inventors conducted further research based on this finding, and accomplished the present invention.

More specifically, the present invention includes the embodiments set out in the appended set of claims.

### Advantageous Effects of Invention

According to the present invention, a compound comprising a chain structure in which a plurality of ubenimex molecules is linked to a polypeptide containing a lysine residue is used to thereby provide a higher anticancer effect than ubenimex; in particular, a higher anticancer effect on solid cancer. Further, a combination of this compound with another anticancer agent can provide a synergistic effect. The compound is also useful as a CD13/APN activity inhibitor and the like.

### Brief Description of Drawings

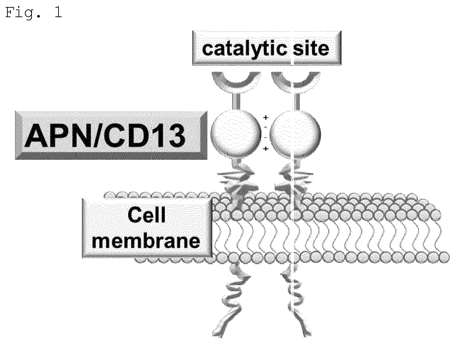

Fig. 1 is a schematic diagram showing the structure of APN/CD13.

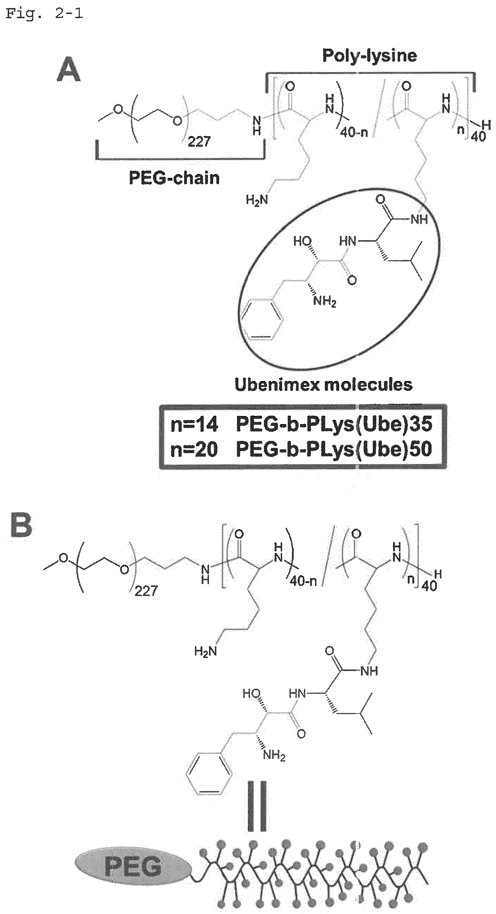

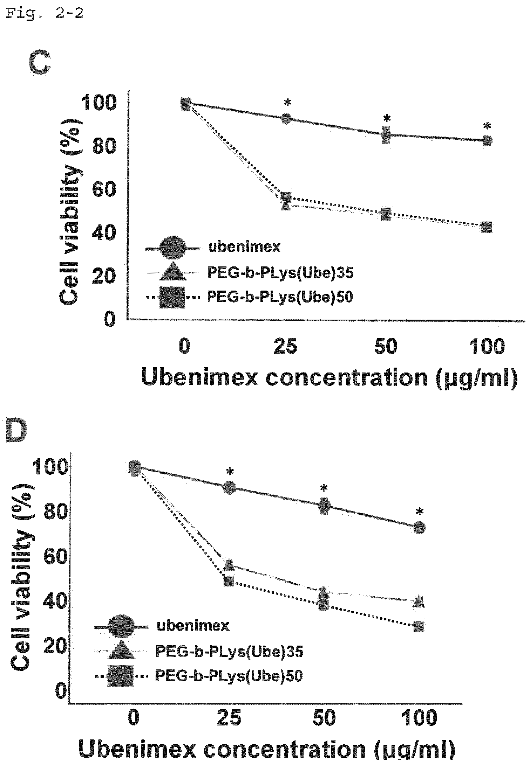

Fig. 2A and Fig. 2B show structural formulas and schematic diagrams of compounds comprising a chain structure in which amino groups on the side chains of some lysine residues of a block copolymer of polyethylene glycol and polylysine (PEG-b-Plys) are linked to carboxy groups of ubenimex molecules by an amide bond (Synthetic Example 1-3). Fig. 2C shows the results of assay using a 3D culture system in Test Example 1. Fig. 2D shows the results of MTT assay in Test Example 2. In Fig. 2C and Fig. 2D, the horizontal axis shows the concentration of the test substance in the medium in terms of ubenimex, and * indicates that the P value is less than 0.05.
Fig. 3A shows the measurement results of the CD13/APN enzyme activity in Test Example 3. In Fig. 3A, * indicates that the P value is less than 0.05. Fig. 3B shows the results of MTT assay in Test Example 4. In Fig. 3B, the horizontal axis shows the concentration of the test substance in medium (PEG-b (-Plys (Ube) 50), and * indicates that the P value is less than 0.05.
Fig. 4A shows the results of ROS level analysis in Test Example 5. Fig. 4B shows the results of apoptosis analysis in Test Example 6. In Fig. 4A and 4B, * shows that the P value is less than 0.05.
Fig. 5A shows the results of isobologram analysis (Test Example 7) using HuH7 cells. Fig. 5B shows the results of isobologram analysis using HepG2 cells.
Fig. 6 shows the results of *in vivo* analysis of antitumor effect (Test Example 8).
Fig. 7 shows the results of pharmacokinetic analysis (Test Example 10).
Fig. 8 shows the results of analysis of the combined effect with another anticancer agent (results of measurement of subcutaneous tumor volume) (Test Example 14).
Fig. 9 shows the results of analysis of the combined effect with another anticancer agent (results of hematoxylin-eosin staining) (Test Example 14).
Fig. 10 shows the results of analysis of the combined effect with another anticancer agent (results of immunohistochemical staining) (Test Example 14).
Fig. 11 shows the results of analysis of the combined effect with another anticancer agent (results of quantification of cell proliferation markers) (Test Example 14)).

### Description of Embodiments

The terms "containing" and "comprising" as used herein include the concepts of "containing," "comprising," "substantially consisting of," and "consisting of."

### 1. Compound

The present invention relates to a compound containing a chain structure in which a plurality of ubenimex molecules is linked to a polypeptide containing a lysine residue (sometimes referred to herein as "the compound of the present invention"). This is explained below.

Ubenimex is a compound represented by the following formula: 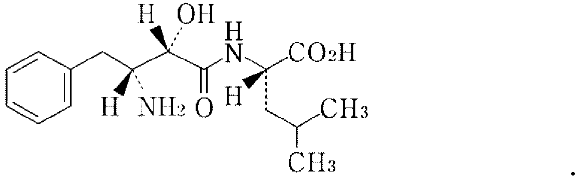 The chemical name of ubenimex is (2S)-2-[(2S,3R)-3-amino-2-hydroxy-4-phenylbutanoylamino]-4-methylpentanoic acid.

To easily link ubenimex, the polypeptide contains an amino functional group of the lysine residue that is reactive with a functional group of ubenimex and may additionally have a functional group that is reactive with a functional group of ubenimex (e.g., a carboxy group, an amino group, or a hydroxy group, aldehyde groups, carbonyl groups, or organic halides). From the viewpoint of easy interaction with a cell membrane that is usually charged negatively, the polypeptide is preferably cationic (for example, amino group, a guanidino group, an imidazole group, or an amidine structure).

The degree of polymerization (integer) of the polypeptide is 5 to 200, preferably 10 to 150, more preferably 20 to 100, even more preferably 30 to 80, and still even more preferably 35 to 50.

Under the provision that the polypeptide contains a lysine residue, the amino acid residue may be a natural amino acid residue, or a synthetic amino acid residue. This means, for example, that an amino acid residue is replaced with an amino acid residue having a similar side chain. Examples include amino acid residues having a basic side chain, such as lysine, arginine, and histidine; amino acid residues having an acidic side chain, such as aspartic acid and glutamic acid; amino acid residues having an uncharged polar side chain, such as glycine, asparagine, glutamine, serine, threonine, tyrosine, and cysteine; amino acid residues having a nonpolar side chain, such as alanine, valine, leucine, isoleucine, proline, phenylalanine, methionine, and tryptophan; amino acid residues having a β-branched side chain, such as threonine, valine, and isoleucine; amino acid residues having an aromatic side chain, such as tyrosine, phenylalanine, tryptophan, and histidine.

Since the polypeptide contains a lysine residue, easy interaction with a cell membrane that is usually charged negatively is achieved.

The proportion of the lysine residue is 90% or more, preferably 95% or more, and more preferably 100%, per 100% in total of the amino acid residues that constitute the polypeptide.

From the viewpoint of easy interaction with a cell membrane that is usually charged negatively, the polypeptide has a smaller proportion of amino acid residues having an acidic side chain. For example, the proportion of such residues is 10% or less, preferably 5% or less, and still even more preferably 0%, per 100% in total of the amino acid residues that constitute the polypeptide.

The chain structure is a structure in which a plurality of ubenimex molecules is linked to a polypeptide containing a lysine residue. Although a restrictive interpretation is not desired, the compound of the present invention has a higher anticancer effect on, in particular, solid cancer, than a monomer having ubenimex linked thereto, presumably because a plurality of ubenimex molecules in the compound is bound to CD13/APN.

The mode of linking the chain polymer and ubenimex is not particularly limited. The linkage is, for example, a bond formed by a reaction between a functional group on the chain polymer (preferably on a side chain thereof) and a functional group on ubenimex; preferably a bond formed by a reaction between a functional group on the chain polymer (preferably on a side chain thereof) and a carboxy group on ubenimex; and more preferably an amide bond between an amino group on the chain polymer (preferably on a side chain thereof) and a carboxy group on ubenimex.

The number of ubenimex molecules linked to the chain polymer is, for example, 2 to 500, preferably 2 to 100, more preferably 5 to 50, even more preferably 5 to 30, and still even more preferably 10 to 25.

The number of ubenimex molecules linked to the chain polymer is not particularly limited; and may be, for example, 5 to 90%, preferably 10 to 80%, more preferably 20 to 70%, even more preferably 30 to 60%, and still even more preferably 30 to 55%, when the total degree of polymerization of the chain polymer is defined as 100%.

The compound of the present invention further contains a polyethylene glycol chain structure linked to an end of the chain structure and may further contain another structure. Examples of this other structure include a structure capable of enhancing blood stability of the compound of the present invention, a structure capable of enhancing accumulation of the compound of the present invention in cancer tissue, and the like. Examples of this other structure further include polymers having a zwitterionic structure in a side chain thereof; aptamers having affinity to cancer cells, peptide molecules, antibodies, and antibody fragments; and combinations thereof.

The average molecular weight of this other structure is not particularly limited; and is, for example, 2000 to 50000, and preferably 5000 to 20000, from the viewpoint of blood stability, accumulation in cancer tissue, etc.

A more specific embodiment of the compound of the present invention is preferably, for example, a compound represented by formula (1): 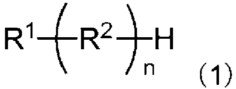
(wherein R¹ represents a polyethylene glycol chain structure; each R² independently represents R²a (a structural unit (monomer) of a polypeptide containing a lysine)or R²b (a structural unit (monomer) of a polypeptide containing a lysine residue and having ubenimex linked thereto), and n represents the degree of polymerization of the chain polymer, which is between 5 and 200); and
particularly a compound represented by formula (1A): 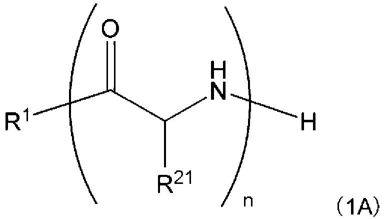
(wherein R¹ represents a polyethylene glycol chain structure, R²¹ in each occurrence independently represents R^{21a} (a side chain of an amino acid residue) or R^{21b} (a side chain of an amino acid residue having ubenimex linked thereto), wherein the proportion of the lysine residue per 100% in total of the amino acid residues that constitute the polypeptide is 90% or more, and n represents the degree of polymerization of the chain polymer, which is between 5 and 200).

Each term defining the formula is as explained above. In the formula, the number of R^{2b} or R^{21b} is not particularly limited; and is, for example, 5 to 90%, preferably 10 to 80%, more preferably 20 to 70%, even more preferably 30 to 60%, and still even more preferably 30 to 55%, relative to the number of n taken as 100%.

The compound of the present invention may be in the form of a salt. The salt is not particularly limited, as long as it is a pharmaceutically acceptable salt. The salt can be an acidic salt or a basic salt. Examples of acidic salts include inorganic acid salts such as hydrochloride, hydrobromide, sulfate, nitrate, and phosphate; organic acid salts such as acetate, propionate, tartrate, fumarate, maleate, citrate, methanesulfonate, and p-toluenesulfonate; amino acid salts such as aspartate and glutamate; and the like. Examples of basic salts include alkali metal salts such as sodium salts and potassium salts; alkaline earth metal salts such as calcium salts and magnesium salts; and the like.

The compound of the present invention may be in the form of a solvate form. The solvent is not particularly limited, as long as it is pharmaceutically acceptable. Examples include water, ethanol, glycerol, acetic acid, and the like.

The compound of the present invention can be synthesized by various methods. For example, the compound can be obtained by reacting ubenimex with either a chain structure, or a structure in which a chain structure and another structure are linked. The type of reaction, reaction conditions, etc. can be appropriately set according to the kind of chain polymer, in particular, the type of functional group of the chain polymer etc.; and further according to the type of functional group on ubenimex to be reacted with the functional group of the chain polymer etc. For example, when the linkage between ubenimex and the chain polymer is an amide bond, the compound represented by formula (1A) among the compounds of the present invention can be synthesized according to the following reaction scheme I: 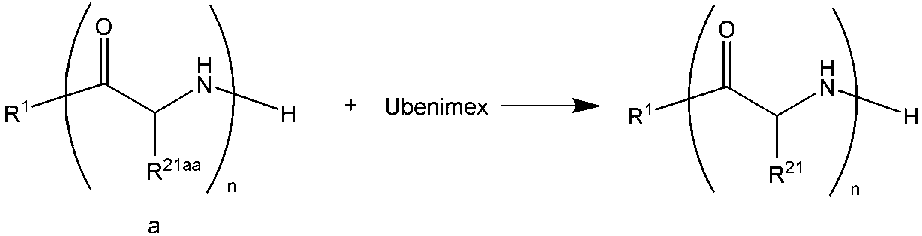 (wherein each symbol other than R^{21aa} is as described above, and R^{21aa} represents a side chain of an amino acid residue having an amino group). Reaction scheme I is described in detail below.

In this reaction, the compound a is reacted with ubenimex to obtain a compound represented by formula (1A). As needed, a protected ubenimex (e.g., a trifluoroacetic acidprotected ubenimex) is preferably used in place of ubenimex.

From the viewpoint of yield etc., in general, the amount of ubenimex and/or protected ubenimex to be used is preferably 0.1 to 2 parts by weight, and more preferably 0.3 to 1.2 parts by weight, per part by weight of the compound a.

This reaction is preferably performed in the presence of a condensing agent. The condensing agent is not particularly limited. For example, a wide variety of known condensing agents can be used. The condensing agent is preferably a triazine condensing agent such as DMT-MM (4-(4,6-dimethoxy-1,3,5-triazin-2-yl)-4-methylmorpholinium chloride n-hydrate). Such condensing agents can be used singly, or in a combination of two or more.

The amount of condensing agent to be used varies depending on the type of the condensing agent. For example, the condensing agent is preferably used in an amount of 0.7 to 1.5 moles, and more preferably 1.0 to 1.2 moles, per mole of ubenimex and/or protected ubenimex.

This reaction is usually performed in the presence of a reaction solvent. The reaction solvent is not particularly limited, and examples include water and the like. Such solvents can be used singly, or in a combination of two or more. It is preferable to add a buffer, such as a carbonate buffer, to the solvent.

As for the reaction temperature, the reaction can be performed with heating, at room temperature, or with cooling. It is usually preferable that the reaction be performed at 0 to 50°C (in particular, 10 to 30°C). The reaction time is not particularly limited; and can be usually 4 hours to 48 hours, and particularly 8 hours to 24 hours.

The progress of the reaction can be tracked by a usual method, such as chromatography. After completion of the reaction, if necessary, a deprotection treatment is performed, and the solvent is then distilled off. The resulting product can be isolated and purified by a usual method, such as chromatography or recrystallization. The structure of the obtained product can be identified by elemental analysis, MS (FD-MS) analysis, IR analysis, ¹H-NMR, ¹³C-NMR, or the like.

### 2. Use

The compound of the present invention has a cell growth inhibitory effect, an apoptosis-promoting effect, a CD13/APN enzyme activity inhibitory effect, an intracellular reactive oxygen species-enhancing effect, and the like. Therefore, the compound of the present invention is more specifically used as an active ingredient of medicaments, reagents, and the like (also referred to herein as "pharmaceutical agents of the present invention"); more specifically, as an active ingredient of anticancer agents, cell growth inhibitors, apoptosis promoters, CD13/APN enzyme activity inhibitors, intracellular reactive oxygen species enhancers, and the like.

The pharmaceutical agent of the present invention is not particularly limited as long as it contains the compound of the present invention, and may further contain one or more other components as necessary. These other components are not particularly limited, as long as they are pharmaceutically acceptable components. Examples of such other components include components having a pharmacological action, and additives. Examples of additives include bases, carriers, solvents, dispersants, emulsifiers, buffers, stabilizers, excipients, binders, disintegrants, lubricants, thickeners, humectants, colorants, fragrances, chelating agents, and the like.

The compound of the present invention alone can exert the above-mentioned effect. Therefore, the pharmaceutical agent of the present invention can exhibit its desired effect without containing any other component having the above-mentioned effects and/or actions, but may contain one or more other components having a pharmacological action.

The compound of the present invention can be used in combination with other anticancer agents. This combination can provide an enhanced effect. Other anticancer agents are not particularly limited, and various anticancer agents can be used. Examples of anticancer agents include alkylating agents, metabolic antagonists, microtubule inhibitors, antibiotic anticancer agents, topoisomerase inhibitors, platinum drugs, molecular targeted drugs, hormone agents, biological agents, and the like. Preferable examples include metabolic antagonists, antibiotic anticancer agents, platinum drugs, and the like.

Examples of alkylating agents include cyclophosphamide, ifosfamide, nitrosourea, dacarbazine, temozolomide, nimustine, busulfan, melphalan, procarbazine, ranimustine, and the like.

Examples of metabolic antagonists include enocitabine, carmofur, capecitabine, tegafur, tegafur-uracil, tegafurgimeracil-oteracil potassium, gemcitabine, cytarabine, cytarabine ocfosfate, nelarabine, fluorouracil, fludarabine, pemetrexed, pentostatin, methotrexate, cladribine, doxifluridine, hydroxycarbamide, mercaptopurine, and the like.

Examples of microtubule inhibitors include alkaloid anticancer agents such as vincristine; and taxane anticancer agents such as docetaxel and paclitaxel.

Examples of antibiotic anticancer agents include mitomycin C, doxorubicin, epirubicin, daunorubicin, bleomycin, actinomycin D, aclarubicin, idarubicin, pirarubicin, peplomycin, mitoxantrone, amrubicin, zinostatin stimalamer, and the like.

Examples of topoisomerase inhibitors include CPT-11, irinotecan, and nogitecan, which have topoisomerase I inhibitory action; and etoposide and sobuzoxane, which have topoisomerase II inhibitory action.

Examples of platinum drugs include cisplatin, nedaplatin, oxaliplatin, carboplatin, and the like.

Examples of hormone agents include dexamethasone, finasteride, tamoxifen, astrozole, exemestane, ethinylestradiol, chlormadinone, goserelin, bicalutamide, flutamide, prednisolone, leuprorelin, letrozole, estramustine, toremifene, fosfestrol, mitotane, methyltestosterone, medroxyprogesterone, mepitiostane, and the like.

Examples of biological drugs include interferon α, interferon β, interferon γ, interleukin 2, ubenimex, dry BCG, and the like.

Examples of molecular targeted drugs include rituximab, alemtuzumab, trastuzumab, cetuximab, panitumumab, imatinib, dasatinib, nilotinib, gefitinib, erlotinib, temsirolimus, bevacizumab, VEGF trap, sunitinib, sorafenib, tosituzumab, bortezomib, gemutuzumab-ozogamicin, ibritumomab-ozogamicin, ibritumomab tiuxetan, tamibarotene, tretinoin, and the like. In addition to the above specified molecular targeted drugs, examples include the following molecular targeted drugs: angiogenesis-targeted inhibitors such as human epidermal growth factor receptor 2 inhibitors, epidermal growth factor receptor inhibitors, Bcr-Abl tyrosine kinase inhibitors, epidermal growth factor tyrosine kinase inhibitors, mTOR inhibitors, and endothelial growth factor receptor 2 inhibitors (α-VEGFR-2 antibodies); various tyrosine kinase inhibitors such as MAP kinase inhibitors; cytokine-targeted inhibitors; proteasome inhibitors; and antibody-anticancer agent complexes. These inhibitors also include corresponding antibodies.

The mode of using the pharmaceutical agent of the present invention is not particularly limited. An appropriate mode of use can be selected according to the type of pharmaceutical agent. The pharmaceutical agent of the present invention can be used, for example, *in vitro* (for example, added to a culture medium of cultured cells) or *in vivo* (for example, administered to an animal), according to the purpose of use.

The target for application of the pharmaceutical agent of the present invention is not particularly limited. Examples of target mammals include humans, monkeys, mice, rats, dogs, cats, rabbits, pigs, horses, bovine, sheep, goats, and deer. Examples of cells include animal cells and the like. The kind of cell is also not particularly limited. Examples of cells include blood cells, hematopoietic stem cells/progenitor cells, gametes (spermatozoa, oocytes), fibroblasts, epithelial cells, vascular endothelial cells, nerve cells, hepatocytes, keratinocytes, muscle cells, epidermal cells, endocrine cells, ES cells, iPS cells, tissue stem cells, cancer cells, and the like.

When the pharmaceutical agent of the present invention is used as an anticancer agent or applied to cancer cells, the target cancer is not particularly limited. Examples include hepatocellular carcinoma, pancreatic cancer, kidney cancer, leukemia, esophageal cancer, gastric cancer, colorectal cancer, lung cancer, prostate cancer, skin cancer, breast cancer, cervical cancer, and the like. Among these, the target cancer is preferably a solid cancer, and more preferably hepatocellular carcinoma.

The pharmaceutical agent of the present invention can be in any dosage form. Examples of dosage forms include oral dosage forms, such as tablets (e.g., orally disintegrating tablets, chewable tablets, effervescent tablets, lozenges, and jelly-like drops), pills, granules, fine granules, powders, hard capsules, soft capsules, dry syrups, liquids (including health drinks, suspensions, and syrups), and jelly formulations; and parenteral dosage forms, such as injectable formulations (e.g., drip infusions (e.g., formulations for intravenous drip infusion), intravenous injections, intramuscular injections, subcutaneous injections, and intradermal injections), topical agents (e.g., ointments, plasters, and lotions), suppositories, inhalants, ophthalmic formulations, ophthalmic ointments, nasal drops, ear drops, and liposome formulations.

The administration route of the pharmaceutical agent of the present invention is not particularly limited, as long as the desired effect can be obtained. Examples include oral administration; parenteral administration including enteral administration, such as tube-feeding and enema administration, intravenous administration, intraarterial administration, intramuscular administration, intracardiac administration, subcutaneous administration, intradermal administration, and intraperitoneal administration; and the like.

The content of the active ingredient in the pharmaceutical agent of the present invention varies depending on, for example, the mode of use, the target of application, the condition of the target, etc.; and is not limited. For example, the content of the active ingredient is 0.0001 to 100 wt.%, and preferably 0.001 to 50 wt.%.

The dosage of the pharmaceutical agent of the present invention in the case of administration to an animal is not particularly limited, as long as it is a pharmaceutically effective amount. In the case of oral administration, in general, the dosage, in terms of the weight of the active ingredient, is usually 0.1 to 1000 mg/kg of body weight per day, and preferably 0.5 to 500 mg/kg of body weight per day. In the case of parenteral administration, the dosage is 0.01 to 100 mg/kg of body weight per day, and preferably 0.05 to 50 mg/kg of body weight per day. The above dosage can also be increased or decreased as appropriate depending on the age, disease state, symptoms, etc.

### Examples

The present invention is described below in detail with reference to Examples. However, the present invention is not limited by the Examples.

### Synthesis Example 1: Synthesis of PEG-b-Plys (Ube) 20

Compounds each comprising a chain structure in which carboxy groups of ubenimex molecules were linked to amino groups on side chains of 20% of lysine residues of a block copolymer of polyethylene glycol and polylysine (PEG-b-Plys) by an amide bond were produced. More specifically, compounds represented by the structural formulas shown in Figs. 2A and 2B wherein n=8 were synthesized in the following manner.

### Synthesis Example 1-1: Synthesis of PEG-b-Plys

1.1 g of polyethylene glycol (PEG) having a methoxy group at one end and an amino group at the other end (average molecular weight: 10000) was weighed out, and dissolved in 10 mL of dimethyl sulfoxide. 1.3 g of N-epsilon-trifluoroacetyl-L-lysine-N-carboxy anhydride (Lys(TFA)-NCA) was dissolved in 10 mL of dimethyl sulfoxide. The two obtained solutions were mixed under an argon atmosphere, and stirred at room temperature overnight. Subsequently, the reaction solution was poured into an excess amount of diethyl ether to reprecipitate and collect the product. The obtained product was dried under reduced pressure. The obtained white powder was dissolved in 100 mL of a mixed solution of methanol/1M NaOH aqueous solution (9/1 [v/v]) and the resulting solution was stirred at 35°C overnight. The reaction solution was neutralized with hydrochloric acid to pH 1 to 2. The resulting mixture was further subjected to dialysis treatment, and freeze-dried to obtain a white powder (1.1 g, yield: 61%). The structure was confirmed by ¹H NMR analysis. The white powder was confirmed to be PEG-b-Plys with an average degree of polymerization of lysine chains of 40 (D₂O, internal standard TSPA, δ (ppm): 1.3-1.9 (240H, m, CO-CH-CH₂-CH₂-CH₂-CH₂-CH₂-NH₂), 3.0 (80H, m, CO-CH-CH₂-CH₂-CH₂-CH₂-NH₂), 3.6 to 3.8 (912H, m, CH₃-O-(CH₂-CH₂-O)-CH₂-CH₂-CH2), 4.3 (40H, m, CO-CH-CH₂-CH₂-CH₂-CH₂-NH₂ )).

### Synthesis Example 1-2: Synthesis of Trifluoroacetic Acid-protected Ubenimex

After 1.5 g of ubenimex was dissolved in 15 mL of methanol, 900 mg of triethylamine and 1350 mg of ethyl trifluoroacetate were added to the obtained solution. The resulting mixture was stirred at room temperature for 2 days. Subsequently, the liquid component was removed under reduced pressure, and re-precipitated in 10 mL of hydrochloric acid (0.01 M). The obtained white powder was dried under reduced pressure to obtain a desired product (1.8 g, 92%).
¹H NMR analysis was performed, and confirmed that the obtained powder had the desired structure (MeOD, internal standard TMS, δ (ppm) : 0.9 (6H, d, (CH₃)₂-CH-CH₂) , 1.3 (1H, m, (CH₃) ₂-CH-CH₂-CH-CH-COOH), 1.6 (2 H, t, (CH₃)₂-CH-CH₂-CH-COOH), 2.9-3.1 (2H, m, C₆H₅-CH₂-CH-CH-OH), 4.1 (1H, d, C₆H₅-CH₂-CH-CH-OH), 4.5 (1H, m, C₆H₅-CH₂-CH-CH-OH), 4.6 (1H, t, (CH₃)₂-CH-CH₂-CH-COOH), 7.1-7.3 (5H, m, C₆H₅-CH₂-CH-CH-OH)) .

### Synthesis Example 1-3: Synthesis of PEG-b-Plys (Ube) 20

After 300 mg of PEG-b-Plys was dissolved in 20 mL of carbonate buffer (50 mM, pH 7.4), 160 mg of trifluoroacetic acidprotected ubenimex and 125 mg of DMT-MM were added. The resulting mixture was stirred at room temperature overnight. Further, the obtained aqueous solution was dialyzed with pure water, and freeze-dried to obtain a white powder. Subsequently, the obtained white powder was dissolved in 10 mL of a mixed solution of methanol and water (1/2 [v/v]), and 130 mg of potassium carbonate was added. The resulting mixture was then allowed to stand at 37°C for 3 days. The obtained solution was dialyzed with pure water, and then freeze-dried to obtain a white powder (280 mg, yield 76%).
¹H NMR analysis was performed, and confirmed that the obtained powder had a structure in which ubenimex was introduced into 20% of lysine side chains of PEG-b-Plys (PEG-b-Plys (Ube) 20) (MeOD, internal standard TMS, δ (ppm): 0.9 (48H, m, (CH₃)₂-CH-CH₂-CH-CONH), 1.3-2.3 (264H, m, (CH₃)₂-CH-CH₂-CH-CONH, CO-CH-CH₂-CH₂-CH₂-CH₂-NHCO), 2.9-3.2 (96H, m, C₆H₅-CH₂-CH-CH-OH, CO-CH-CH₂-CH₂-CH₂-CH₂-CH₂-NHCO), 3.6-3.8 (912H, m , CH₃-O-(CH₂-CH₂-O)-CH₂-CH₂-CH₂), 3.9-4.7 (64H, m, C₆H₅-CH₂-CH-CH-OH, C₆H₅-CH₂-CH-CH-OH, (CH₃)₂-CH-CH₂-CH-CONH, CO-CH-CH₂-CH₂-CH₂-CH₂-NHCO), 7.1-7.3 (40H, m, C₆H₅-CH₂-CH-CH-OH)).

### Synthesis Example 2: Synthesis of PEG-b-Plys (Ube) 35

Compounds each comprising a chain structure in which carboxy groups of ubenimex molecules were linked to amino groups on the side chains of 35% of lysine residues of PEG-b-Plys by an amide bond were produced. More specifically, compounds represented by the structural formulas of Fig. 2A and 2B wherein n=14 were synthesized in the following manner.

100 mg of PEG-b-Plys was dissolved in 10 mL of carbonate buffer (50 mM, pH 7.4). 54 mg of trifluoroacetic acidprotected ubenimex and 41 mg of DMT-MM were added. The resulting mixture was stirred at room temperature overnight. Further, the obtained aqueous solution was dialyzed with pure water, and freeze-dried to obtain a white powder. Subsequently, the obtained white powder was dissolved in 5 mL of a mixed solution of methanol/water (1/2 [v/v]). After adding 46 mg of potassium carbonate, the resulting mixture was stirred at 40°C for 2 days. The obtained solution was dialyzed with pure water, and then freeze-dried to obtain a white powder (80 mg, yield 63%).
¹H NMR analysis was performed, and confirmed that the obtained powder had a structure in which ubenimex was introduced into 35% of lysine side chains of PEG-b-Plys (PEG-b-Plys (Ube) 35) (MeOD, internal standard TMS, δ (ppm): 0.9 (84H, m, (CH₃)₂-CH-CH₂-CH-CONH), 1.3-2.3 (282H, m, (CH₃)₂-CH-CH₂-CH-CONH, CO-CH-CH₂-CH₂-CH₂-CH₂-NHCO), 2.9-3.2 (108H, m, C₆H₅-CH₂-CH-CH-OH, CO-CH-CH₂-CH₂-CH₂-CH₂-NHCO), 3.6-3.8 (912H, m, CH₃-O-(CH₂-CH₂-O)-CH₂-CH₂-CH₂), 3.9-4.7 (82H, m, C₆H₅-CH₂-CH-CH-OH, C₆H₅-CH₂-CH-CH-OH, (CH₃)₂-CH-CH₂-CH-CONH, CO-CH-CH₂-CH₂-CH₂-CH₂-NHCO), 7.1-7.3 (70H, m, C₆H₅-CH₂-CH-CH-OH)).

### Synthesis Example 3: Synthesis of PEG-b-Plys (Ube) 50

Compounds each comprising a chain structure in which carboxy groups of ubenimex molecules were linked to amino groups on 50% of lysine residue side chains of PEG-b-Plys by an amide bond was synthesized. More specifically, compounds shown in Figs. 2A and 2B wherein n=20 were synthesized in the following manner.

After 300 mg of PEG-b-Plys was dissolved in 20 mL of carbonate buffer (50 mM, pH 7.4), 320 mg of trifluoroacetic acidprotected ubenimex and 250 mg of DMT-MM were added. The resulting mixture was stirred at room temperature overnight. Further, the obtained aqueous solution was dialyzed with pure water, and freeze-dried to obtain a white powder. Subsequently, the obtained white powder was dissolved in 10 mL of a mixed solution of methanol/water (1/2 [v/v]). After 260 mg of potassium carbonate was added, the resulting mixture was allowed to stand at 37°C for 3 days. The obtained solution was dialyzed against pure water, and then freeze-dried to obtain a white powder (360 mg, yield: 81%).
¹H NMR analysis was performed, and confirmed that the obtained power had a structure in which ubenimex was introduced into 50% of lysine side chains of PEG-b-Plys (PEG-b-Plys (Ube) 50) (MeOD, internal standard TMS, δ (ppm): 0.9 (120H, m, (CH₃)₂-CH-CH₂-CH-CONH), 1.3-2.3 (300H, m, (CH₃)₂-CH-CH₂-CH-CONH, CO-CH-CH₂-CH₂-CH₂-CH₂-NHCO), 2.9-3.2 (120H, m, C₆H₅-CH₂-CH-CH-OH, CO-CH-CH₂-CH₂-CH₂-CH₂-CH₂-NHCO), 3.6-3.8 (912H, m, CaH₃-O-(CH₂-CH₂-O)-CH₂-CH₂-CH₂), 3.9-4.7 (100H, m, C₆H₅-CH₂-CH-CH-OH, C₆H₅-CH₂-CH-CH-OH, (CH₃)₂-CH-CH₂-CH-CONH, CO-CH-CH₂-CH₂-CH₂-CH₂-NHCO), 7.1-7.3 (100H, m, C₆H₅-CH₂-CH-CH-OH)).

### Test Example 1: Evaluation of Antitumor Effect Using 3D Culture System

Cells were cultured using Dulbecco's modified Eagle's medium (DMEM) containing 500 ug/ml of antibiotic penicillinstreptomycin and 10% FBS (hereafter referred to as complete medium) in an incubator adjusted to 37°C, 5% CO₂ (the same applies to the following Test Examples). For 3D culture, 8 x 10³ HuH7 cells were seeded in a 96-well Cell-able (registered trademark, produced by Toyo Gosei Co., Ltd.), and cultured at 37°C for 3 days. After confirming under a microscope that hemispheres were formed almost uniformly on the wells, a test substance (ubenimex (free ubenimex), PEG-b-Plys (Ube) 35, or PEG-b-Plys (Ube) 50) was added to each well, and cultured at 37°C for another 2 days. The cells were then stained with a DAPI solution for 30 minutes. The absorbance of the plate was measured at 570 nm and then at 650 nm using a microplate reader. The results are shown in terms of percentage absorbance relative to the untreated control.

Fig. 2C shows the results. As shown in Fig. 2C, PEG-b-Plys (Ube) 35 or PEG-b-Plys (Ube) 50 was confirmed to have a significantly higher antitumor effect at a lower concentration than ubenimex.

### Test Example 2: Evaluation of Antitumor Effect by MTT Assay

HuH7 cells were cultured in medium containing a test substance (ubenimex, PEG-b-Plys (Ube) 35, or PEG-b-Plys (Ube) 50) using a 96-well culture plate. After culturing for 72 hours, 10 µl (50 µg) of MTT was added to each well, and culturing was performed at 37°C for 4 hours. Subsequently, the medium was removed, and 100 µl of acidic isopropanol was added to dissolve formazan crystals. The resulting solution was gently shaken for 15 minutes using a microplate shaker. The absorbance of the plate was measured at 570 nm and then at 650 nm using a microplate reader. The results were shown in terms of percentage absorbance relative to the untreated control.

Fig. 2D shows the results. The same results as in Fig. 2C were obtained in the evaluation by MMT assay.

### Test Example 3: Evaluation of Effects on CD13/APN Enzyme Activity

CD13/APN enzyme activity was measured spectrophotometrically using L-leucine-p-nitroanilide (produced by Peptide Institute, Inc.), which is a substrate of CD13/APN. An HepG2 cell suspension at a concentration of 5 x 10⁵ cells in 200 µl of PBS was seeded in each well of a 96-well plate. The above-mentioned substrate was then added to achieve a final concentration of 1.6 mM. Further, a test substance (ubenimex or PEG-b-Plys (Ube) 50) was added to achieve a final concentration of 100 ug/ml in terms of ubenimex. After culturing at 37°C for 60 minutes, CD13/APN enzyme activity was evaluated by measuring the absorbance at 405 nm using a microplate reader (Perkin Elmer EnSpire 2300 Multimode Plate Reader).

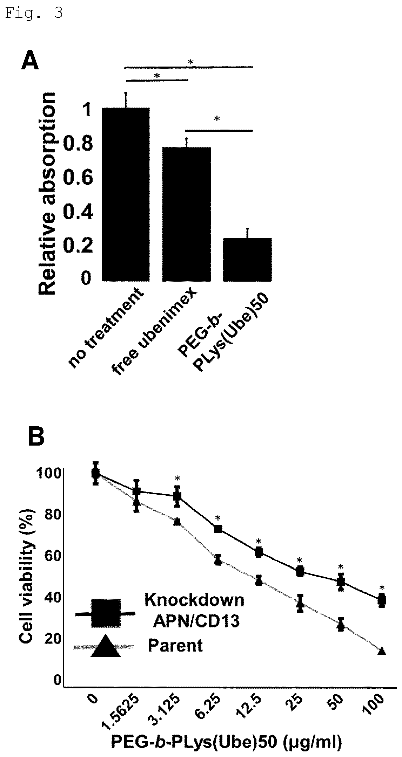

Fig. 3A shows the results. As shown in Fig. 3A, PEG-b-Plys (Ube) 50 was found to more significantly suppress CD13/APN enzyme activity than ubenimex.

### Test Example 4: Analysis of Effect of CD13/APN Knockdown on AntiTumor Effect

Two shRNAs (sh1: SEQ ID NO: 1 and sh2: SEQ ID NO: 2) targeting CD13/APN were cloned into the lentiviral vector pLKO. The vector was co-transfected into HuH7 cells with an expression vector containing gag/pol, rev, and vg genes. Forty-eight hours after transfection, the lentivirus was harvested, and 5 ug/mL polybrene was added. HuH7 cells were infected with the harvested lentivirus, and selected with 1 µg/mL puromycin for 2 weeks. The CD13/APN expression levels of the obtained cells were confirmed by quantitative RT-PCT and Western blotting, and CD13/APN was confirmed to be knocked down. MTT assay was performed in the same manner as in Test Example 2 using CD13/APN knockdown cells and their parent line, HuH7 cells (Parent).

Fig. 3B shows the results. As shown in Fig. 3B, the antitumor effect of PEG-b-Plys (Ube) 50 was found to be attenuated by CD13/APN knockdown. This result and the results of Test Example 3 (Fig. 3A) suggest that the antitumor effect of PEG-b-Plys (Ube) 50 is exerted through inhibition of CD13/APN enzyme activity.

### Test Example 5: Analysis of Effects on Intracellular Reactive Oxygen Species (ROS) Levels

CellROX Deep Red Reagent was purchased from Invitrogen (Carlsbad, CA), and intracellular ROS levels were measured. HepG2 cells were treated with a test substance (ubenimex or PEG-b-Plys (Ube) 50) at 100 ug/ml. The treated cells were cultured at 37°C for 6 hours, and the cell concentration of the sample was adjusted to 5×10³ cells/ml. Then, the cells were cultured with a CellROX Deep Red Reagent (1 mM, Invitrogen) at 37°C for 30 minutes while protected from light. Furthermore, the cells were stained with SYTOX Blue Dead Cell Stain (5 mM, Invitrogen), and counted by flow cytometry after dead cell exclusion. Flow cytometric analysis was performed using a Canto II flow cytometer (BD Biosciences).

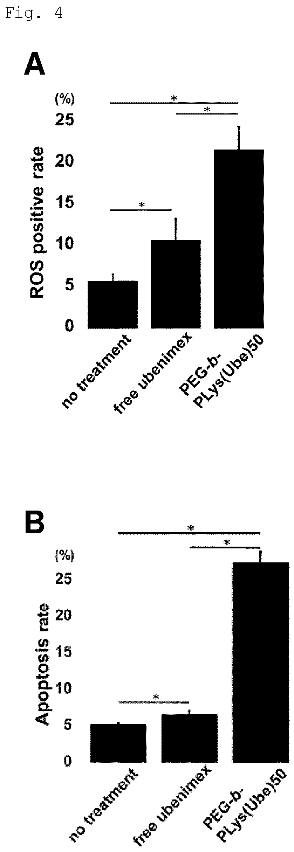

Fig. 4A shows the results. As shown in Fig. 4A, it was found that ubenimex has an effect of enhancing intracellular ROS levels, and that PEG-b-Plys (Ube) 50 is significantly superior to ubenimex in this effect.

### Test Example 6: Analysis of Effects on Apoptosis

HepG2 cells were treated with a test substance (Ubenimex or PEG-b-Plys (Ube) 50) in the same manner as in Test Example 5. The cell apoptosis assay was performed by flow cytometry using an Annexin V-FITC Apoptosis Detection Kit (BioVision, Mountain View, CA) according to the manufacturer's protocol. For flow cytometric analysis, HepG2 cells (5 x 10⁵ cells) were trypsinized, gently washed twice with PBS, and subsequently resuspended in 500 µL of 1 x binding buffer. The cells were stained by adding 5 µL of Annexin V-FITC and propidium iodide (PI) in the dark at room temperature for 5 minutes. The green fluorescence of FITC-bound Annexin V and the red fluorescence of DNA-bound PI were measured using a Canto II flow cytometer (BD Biosciences).

Fig. 4B shows the results. As shown in Fig. 4B, it was found that ubenimex has an effect of inducing apoptosis, and that PEG-b-Plys (Ube) 50 is significantly superior to ubenimex in this effect.

### Test Example 7: Analysis 1 of Combined Effect with Another AntiCancer Agent

PEG-b-Plys (Ube) 50 at various concentrations was combined with another anticancer agent (5-FU, CDDP, or DXR) at various concentrations. Using these combinations as test substances and using HuH7 cells or HepG2 cells, MTT assay was performed in the same manner as in Test Example 2. Based on the obtained results, isobologram analysis was performed. The combination index (CI) was calculated as a means of analyzing combined effects using median effect plot analysis. CI was calculated according to the formula [CI = (dA/D30A) + (dB/D30B)]. In the formula, D30A represents the concentration of drug A (PEG-b-Plys (Ube) 50) required to produce 30% of the effect; and dA represents the concentration of drug A required to produce 30% of the effect when drug A is combined with dB. Similarly, D30B represents the concentration of drug B (another anticancer agent) required to produce 30% of the effect; and dB represents the concentration of drug B required to produce 30% of the effect when drug B is combined with dA. CI values were defined as follows:
<0.8 = having a synergistic effect;
0.8 to 1.2 = having an additive effect; and
>1.2 = having an antagonistic effect.

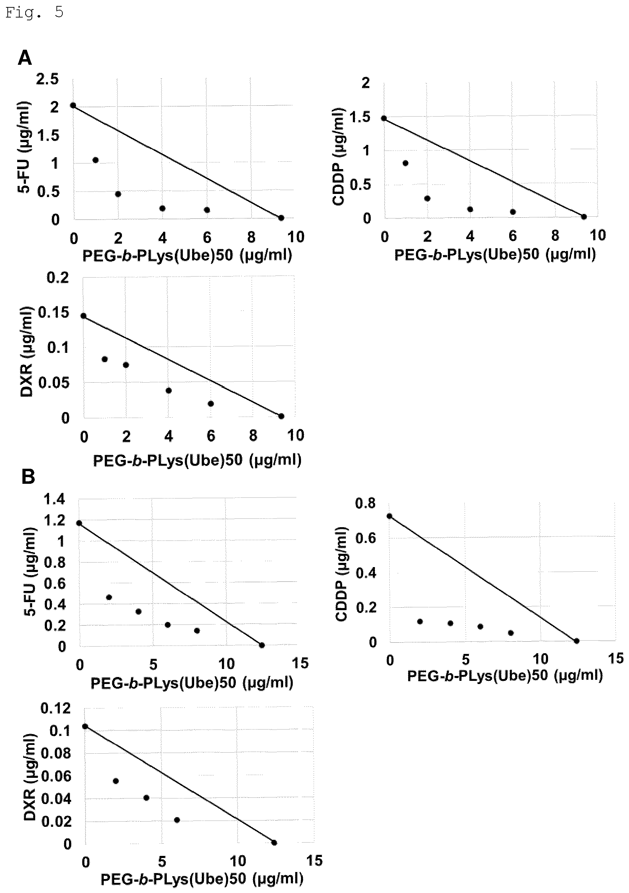

Figs. 5A and 5B show the results. The results shows that the calculated CIs were all less than 0.8. This indicates that PEG-b-Plys (Ube) 50 provides a synergistic effect when used in combination with another anticancer agent (5-FU, CDDP, or DXR) .

### Test Example 8: In vivo Analysis of Antitumor Effect

Eight-week-old NOD/SCID mice were purchased from CLEA Japan, and reared in a pathogen-free environment. HuH7 cells (5 x 10⁶ cells) were mixed with 50 µL of PBS and 50 µL of Matrigel (BD Biosciences), and subcutaneously implanted into the back of the mice. After the subcutaneous tumor volume reached 100 mm³, one of PBS, PEG-b-Plys, and PEG-b-Plys (Ube) 50 was intraperitoneally administered every other day. The subcutaneous tumor volume was calculated as (maximum diameter) x (shortest diameter)² / 2. In all groups, the dose was set at 100 µL, and the concentration of PEG-b-Plys (Ube) 50 was set at 1 mg/ml. The concentration of PEG-b-Plys was adjusted to the same concentration as that of PEG-b-Plys contained in a 1 mg/ml PEG-b-Plys (Ube) 50 solution. Mice in the PEG-b-Plys-treated group and those in the PEG-b-Plys (Ube) 50-treated group were treated until day 21 after the start of administration, and euthanized on day 24. The mice in the PBS-treated group were initially scheduled to be euthanized after the treatment in the same manner as those in the PEG-b-Plys-treated group and the PEG-b-Plys (Ube) 50-treated groups. However, since the tumor in one of the mice exceeded 2 cm, the timing of euthanasia had to be hastened from an ethical standpoint. Thus, the mice were euthanized on day 18 after the start of administration.

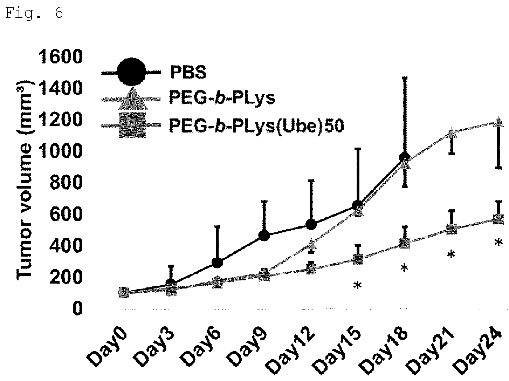

Fig. 6 shows the results. As shown in Fig. 6, it was found that the administration of PEG-b-Plys (Ube) 50 significantly reduces the tumor size.

### Test Example 9: Analysis of Membrane Permeability

PEG-b-Plys (Ube) 50 was labeled with Alexa647 to obtain a labeled compound. 10 µL of a DMSO solution of the labeled compound (2.5 mM) and 990 µL of HBSS (pH 6.5) were mixed and vortexed for 10 minutes to obtain a test solution (500 µM labeled compound solution). 300 µL of the test solution was added to Caco-2 cells (cultured in transwells for 21 days), and 1 mL of HBSS with BSA (pH 7.4) was added to the bottom of the wells. The resulting mixture was incubated at 37°C for 2 hours. The liquids in the upper and lower portions of each well were individually collected. 10 µL of the upper sample (S) of the well (+ 140 µL of BSA-containing HBSS), and 150 µL of the lower sample (M) of the well were individually measured for fluorescence (650 nm/668 nm) with a fluorescence plate reader (M1000, produced by Tecan). Based on the obtained fluorescence intensity, the apparent permeability coefficient (Papp) was then calculated.

The results show that the permeability coefficient (Papp (10⁻⁶ cm/sec)) of the labeled compound was less than 0.1, and the labeled compound was found to be almost impermeable through the membrane.

### Test Example 10: CYP Inhibitory Analysis

The composition of the reaction solution is as follows:
PEG-b-Plys (Ube) 50: final concentration: 0.1, 1, or 10 µM;
liver microsomes: final concentration: 0.1 mg protein/mL;
coenzyme (NADPH, G-6-P, MgCl₂);
G6PDH; and
model substrate Mix (for time-dependent inhibition, this component was added after the above components were allowed to react for 60 minutes).

All the above components were mixed (final volume: 200 µL) to obtain a reaction solution. After incubation at 37°C for 20 minutes, 200 µL of acetonitrile was added, and vortexed for 30 seconds. After centrifugation (3500 rpm, 20 minutes), the supernatant was analyzed by LC/MS to calculate the peak area of each model substrate. From the residual amount of each model substrate, CYP inhibitory activity was calculated (the inhibition rate at 10 µM; and the IC₅₀ value, if calculation was possible).

Table 1 shows the results. As shown in Table 1, no inhibitory activity on each CYP species was observed.

**Table 1**

| pra incubation | IC50 (% inhibition of 10µM test compound) | | | | | | | | | | | | | |
|---|---|---|---|---|---|---|---|---|---|---|---|---|---|---|
| | CYP3A4(M) | | CYP3A4(T) | | CYP2C9 | | CYP2D6 | | CYP1A2 | | CYP2C8 | | CYP2C19 | |
| ( - ) | > 10.0µM | 21% | > 10.0µM | 13% | > 10.0µM | 0% | > 10.0µM | 0% | > 10.0µM | 1% | > 10.0µM | 10% | > 10.0µM | 0% |
| ( - ) | \| 9.2µM | 52% | > 10.0µM | 20% | > 10.0µM | 10% | > 10.0µM | 0% | > 10.0µM, | 0% | > 10.0µM | 24% | > 10.0µM | 14% |

### Test Example 11: Pharmacokinetic Analysis

Alexa647-labeled PEG-b-plys (Ube) 50 was dissolved in PBS to a concentration of 1 mg/mL. The Alexa647-labeled PEG-b-Plys (Ube) 50 solution was intraperitoneally administered to C57BL/6JJcl mice (10-20 weeks old) at 3.33 mg/kg (n=5). 0.5, 2, 8, and 48 hours after the administration, blood was collected from the central vein of each mouse, and the heart, lung, liver, kidney, and spleen were then excised. The excised tissues were washed with PBS, and weighed after removal of water. The organ weight (mg) x 4 µL of lysis buffer (produced by Wako Pure Chemical Industries, Ltd.) was added, and the cells were crushed with a multi-bead shocker. After crushing, each sample was centrifuged at 8400 x g at 4°C for 5 minutes. 50 µL of the supernatant was transferred to a 96-well plate (black) and measured for fluorescence (at 610 nm and 670 nm) using a fluorescence plate reader (EnSpire, PerkinElmer). After blood collection, the blood was separated into plasma and blood cell components by centrifugation at 860 x g and 4°C for 15 minutes. The plasma drug concentration was quantified. A calibration curve was prepared by spiking Alexa647-labeled PEG-b-Plys (Ube) 50 into each tissue of untreated mice to quantify the drug in each tissue.

As a result, most of the administered Alexa647-labeled PEG-b-Plys (Ube) 50 disappeared within 48 hours. Alexa 647-labeled PEG-b-Plys (Ube) 50 was most abundantly distributed in the kidney.

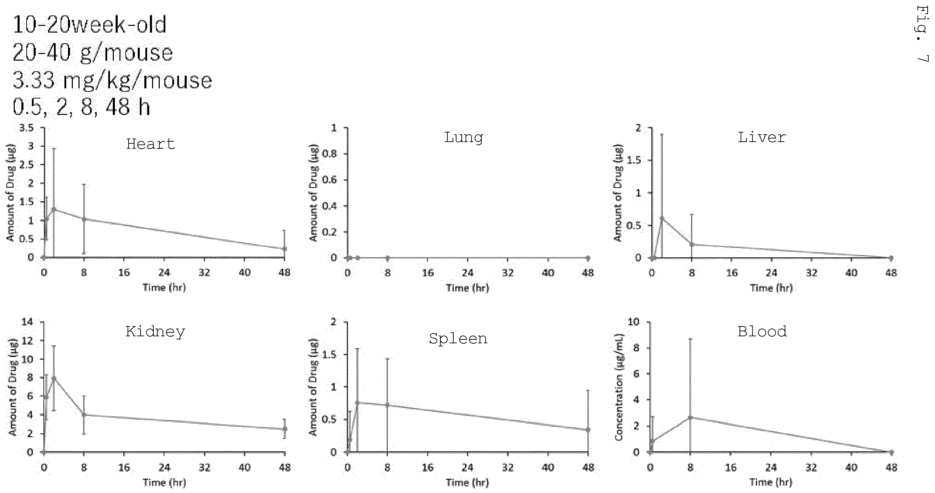

Fig. 7 shows the results.

### Test Example 12: Analysis of Cardiotoxicity

CarmyA-human (Myoridge), which is human iPS cellderived cardiomyocytes, was seeded into a Matrigel-coated 384-well plate (8000 cells/well). The medium was exchanged daily, and cultured for 1 week. As a staining reagent, Cal520 AM (AAT Bioquest, Inc.) was added (final concentration: 5 µM). PEG-b-Plys (Ube) 50 was added (final concentration: 2, 6.7, or 20 µM (n=2)). Before addition of PEG-b-Plys (Ube) 50, and 10 and 30 minutes after the addition, Ca flux assay (using FDSS7000 (Hamamatsu Photonics)) was performed.

As a result, with the addition of the compound, the peak interval tended to widen slightly; however, no tendency to cause QT prolongation or tachycardia was observed.

### Test Example 13: Analysis of Solubility

10 µL of a DMSO solution (10 mM) of the labeled compound obtained in Test Example 9 (10 mM) and 990 µL of PBS were mixed (= 1% DMSO) and vortexed for 10 minutes to prepare a test solution (1% DMSO/PBS solution of the Alexa647-labeled compound (100 µM)). 250 µL of the test solution was filtered through a filtration plate (MultiScreen HTS-PCF), and 200 µL of the obtained filtered sample was measured for fluorescence (at 650 nm and 668 nm) with a fluorescence plate reader (M1000, produced by Tecan). The same operation was performed using a 5% DMSO/PBS solution of the compound as a control. The fluorescence value of each sample was compared with that of the control (100 µM).

As a result, the solubility of PEG-b-Plys (Ube) 50 in PBS was calculated to be 73.7 µM.

### Test Example 14: Analysis 2 of Combined Effect with Another AntiCancer Drug

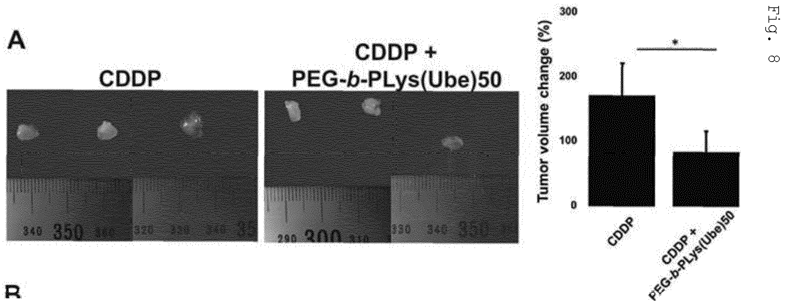

Eight-week-old NOD/SCID mice were purchased from CLEA Japan, and reared in an SPF environment. HuH7 cells (5 x 10⁶ cells) were mixed with 50 µL of PBS and 50 µL of Matrigel (BD Biosciences), and subcutaneously implanted into the back of the mice. After the subcutaneous tumor volume reached 100 mm³, PBS, CDDP, or both of CDDP and PEG-b-Plys (Ube) 50 were intraperitoneally administered every other day. The subcutaneous tumor volume was calculated as (maximum diameter) x (shortest diameter)² / 2. Fig. 8 shows the results.

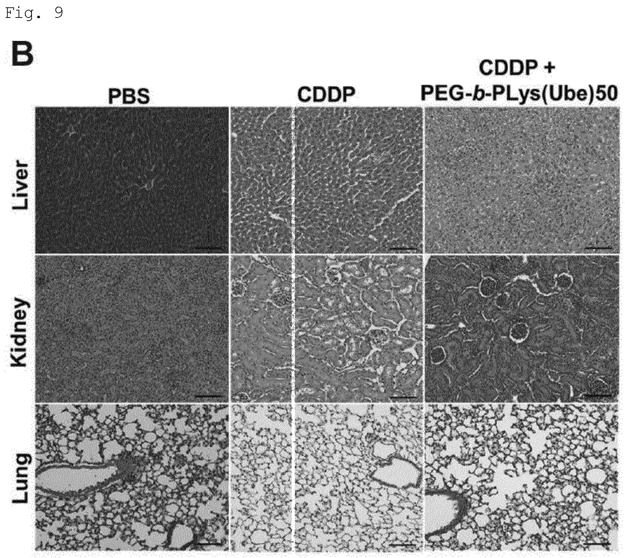

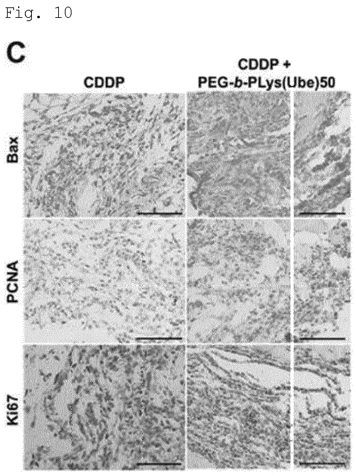

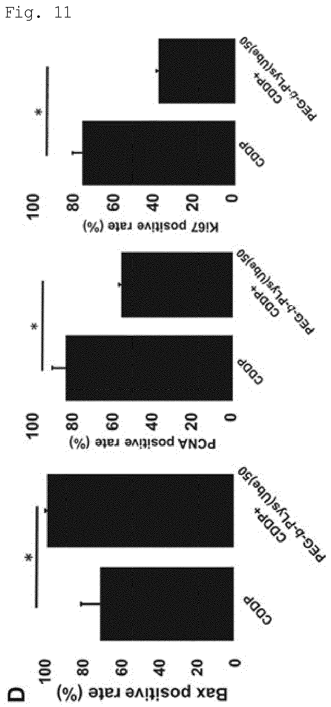

All mouse tissues were fixed by treatment with 10% formaldehyde for 24 hours, embedded in paraffin, and sliced into 5 µm sections. The obtained sections were subjected to hematoxylin-eosin staining and immunohistochemical staining. Anti-Bax antibody (Cat. No. ab32503; Abcam, Cambridge, UK) was used to count Bax-positive cells. Anti-Ki67 (Cat. No. ab15580; Abcam) and anti-PCNA antibody (Cat. No. ab18197; Abcam) were also used similarly. Bax, PCNA, and Ki67 expressions were analyzed in three different random fields of view, and the average percentage of positive cells was evaluated. Figs. 9 to 11 show the results.

The results clearly show that the combination of CDDP with PEG-b-Plys (Ube) 50 significantly reduces the tumor volume compared to CDDP alone; and that *in vivo* as well, PEG-b-Plys (Ube) 50 provides a synergistic effect with an existing anticancer agent. Further, the administration of PEG-b-Plys (Ube) 50 did not provide any clear histologically adverse effect on the liver, kidney, or lung. Bax expression was significantly higher and cell proliferation marker was significantly lower in the PEG-b-Plys (Ube) 50 combination group than in the CDDP alone group.

The references to methods of treatment by therapy or surgery or in vivo diagnosis methods in the examples of this description are to be interpreted as references to compounds, pharmaceutical compositions and medicaments of the present invention for use in those methods.

## Claims

1. A compound comprising a chain structure in which a plurality of ubenimex molecules is linked to a polypeptide containing a lysine residue,
wherein a total of the amino acid residues that constitute the polypeptide is 5 to 200,
wherein the proportion of the lysine residue is 90% or more per 100% in total of the amino acid residues that constitute the polypeptide, and
wherein a polyethylene glycol chain structure is linked to an end of the chain structure.

2. The compound according to claim 1, wherein the linkage is an amide bond formed between an amino group in the polypeptide and a carboxy group of the ubenimex.

3. The compound according to claim 1 or 2, wherein the polypeptide has an average molecular weight of 500 to 30000.

4. The compound according to any one of claims 1 to 3, wherein the number of ubenimex molecules linked to the polypeptide is 2 to 100.

5. The compound according to any one of claims 1 to 4, which is a compound represented by formula (1A): 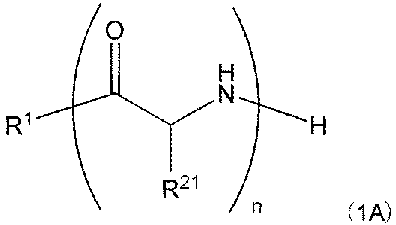
wherein R¹ represents a polyethylene glycol chain structure, R²¹ in each occurrence independently represents R^{21a} (a side chain of a lysine residue) or R^{21b} (a side chain of a lysine residue having ubenimex linked thereto), and n represents 5 to 200);
a salt of the compound;
a solvate of the compound; or
a solvate of a salt of the compound.

6. A medicament comprising the compound of any one of claims 1 to 5.

7. A reagent comprising the compound of any one of claims 1 to 5.

8. An anticancer agent comprising the compound of any one of claims 1 to 5.

9. The anticancer agent according to claim 8, wherein the target cancer is a solid cancer.

10. The anticancer agent according to claim 8 or 9, further comprising another anticancer compound.

11. The anticancer agent according to claim 8 or 9, which is used for administration in combination with another anticancer compound.

12. A CD13/APN activity inhibitor comprising the compound of any one of claims 1 to 5.

## Patentansprüche

1. Verbindung, umfassend eine Kettenstruktur, in der eine Mehrzahl von Ubenimex-Molekülen an ein Polypeptid, enthaltend einen Lysinrest, gebunden ist,
wobei eine Gesamtzahl der Aminosäurereste, aus denen das Polypeptid besteht, 5 bis 200 beträgt,
wobei der Anteil des Lysinrests 90% oder mehr pro 100% der gesamten Aminosäurereste, aus denen das Polypeptid besteht, beträgt, und
wobei eine Polyethylenglykol-Kettenstruktur an ein Ende der Kettenstruktur gebunden ist.

2. Verbindung nach Anspruch 1, wobei die Bindung eine Amidbindung ist, die zwischen einer Aminogruppe im Polypeptid und einer Carboxygruppe des Ubenimex gebildet wird.

3. Verbindung nach Anspruch 1 oder 2, wobei das Polypeptid ein durchschnittliches Molekulargewicht von 500 bis 30000 aufweist.

4. Verbindung nach einem der Ansprüche 1 bis 3, wobei die Anzahl der an das Polypeptid gebundenen Ubenimex-Moleküle 2 bis 100 beträgt.

5. Verbindung nach einem der Ansprüche 1 bis 4, die eine Verbindung der Formel (1A) ist: 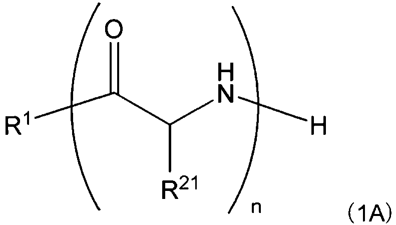
wobei R¹ eine Polyethylenglykol-Kettenstruktur darstellt, R²¹ bei jedem Auftreten unabhängig voneinander R^{21a} (eine Seitenkette eines Lysinrests) oder R^{21b} (eine Seitenkette eines Lysinrests, an den Ubenimex gebunden ist) darstellt und n 5 bis 200 darstellt);
ein Salz der Verbindung;
ein Solvat der Verbindung; oder
ein Solvat eines Salzes der Verbindung.

6. Medikament, umfassend die Verbindung nach einem der Ansprüche 1 bis 5.

7. Reagenz, umfassend die Verbindung nach einem der Ansprüche 1 bis 5.

8. Antitumormittel, umfassend die Verbindung nach einem der Ansprüche 1 bis 5.

9. Antitumormittel nach Anspruch 8, wobei der Zieltumor ein solider Tumor ist.

10. Antitumormittel nach Anspruch 8 oder 9, weiter umfassend eine weitere Antitumorverbindung.

11. Antitumormittel nach Anspruch 8 oder 9, das zur Verabreichung in Kombination mit einer weiteren Antitumorverbindung verwendet wird.

12. CD13/APN-Aktivitätsinhibitor, umfassend die Verbindung nach einem der Ansprüche 1 bis 5.

## Revendications

1. Composé comprenant une structure de chaîne dans laquelle une pluralité de molécules d'ubénimex est liée à un polypeptide contenant un résidu de lysine, dans lequel un total des résidus d'acide aminé qui constituent le polypeptide est de 5 à 200,
dans lequel la proportion du résidu de lysine est de 90 % ou plus pour 100 % au total des résidus d'acide aminé qui constituent le polypeptide, et
dans lequel une structure de chaîne de polyéthylène glycol est liée à une extrémité de la structure de chaîne.

2. Composé selon la revendication 1, dans lequel le lien est une liaison amide formée entre un groupe amino dans le polypeptide et un groupe carboxy de l'ubénimex.

3. Composé selon la revendication 1 ou 2, dans lequel le polypeptide a un poids moléculaire moyen de 500 à 30 000.

4. Composé selon l'une quelconque des revendications 1 à 3, dans lequel le nombre de molécules d'ubénimex liées au polypeptide est de 2 à 100.

5. Composé selon l'une quelconque des revendications 1 à 4, qui est un composé représenté par la formule (1A) : 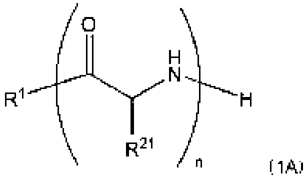
dans laquelle R¹ représente une structure de chaîne de polyéthylène glycol, R²¹ représente indépendamment dans chaque occurrence R^{21a} (une chaîne latérale d'un résidu de lysine) ou R^{21b} (une chaîne latérale d'un résidu de lysine ayant de l'ubénimex lié à elle), et n représente 5 à 200 ;
un sel du composé ;
un solvate du composé ; ou
un solvate d'un sel du composé.

6. Médicament comprenant le composé selon l'une quelconque des revendications 1 à 5.

7. Réactif comprenant le composé selon l'une quelconque des revendications 1 à 5.

8. Agent anticancéreux comprenant le composé selon l'une quelconque des revendications 1 à 5.

9. Agent anticancéreux selon la revendication 8, dans lequel le cancer cible est un cancer solide.

10. Agent anticancéreux selon la revendication 8 ou 9, comprenant en outre un autre composé anticancéreux.

11. Agent anticancéreux selon la revendication 8 ou 9, qui est utilisé pour l'administration en combinaison avec un autre composé anticancéreux.

12. Inhibiteur d'activité CD13/APN comprenant le composé selon l'une quelconque des revendications 1 à 5.
